# EUROPEAN PATENT APPLICATION

(11) **EP 2 305 323 A2**
(43) Date of publication of application: **06.04.2011**
(21) Application number: 10015801.3
(22) Date of filing: 15.11.2007
(51) Int. Cl.: A61L 31/10, A61L 31/16

(54) **Stent with differential timing of abluminal and luminal release of a therapeutic agent**

(30) Priority: 16.11.2006 US 859977 P
(62) Divisional of application: 07867470.2
(71) Applicant: Boston Scientific Limited, Christ Church (BB)
(72) Inventor: Shippy, James, Lee, Wilmington North Carolina 28411 (US); Robertson, Kimberly, A., Forest Lake MN 55025 (US); Weber, Jan, 6228 GJ Maastricht (NL)
(74) Representative: Schildberg, Peter

(57) **Abstract**

A stent designed for implantation into a blood vessel of a patient comprising:
a tubular stent sidewall structure comprising a plurality of struts and openings in the sidewall structure; wherein at least one strut comprises an abluminal surface, a luminal surface opposite the abluminal surface, and side surfaces extending between the luminal and abluminal surfaces;
a first coating composition disposed on the abluminal surface and luminal surface;
and
a second coating composition disposed on a portion of the first coating composition; characterized in that
the portion is disposed either
only on the abluminal surface and side surfaces or
only on the abluminal surface; and
when the stent is implanted, the second coating composition disposed on the abluminal surface faces the blood vessel.

## Description

### Field Of The Invention

The invention relates generally to a medical device that is useful for delivering a therapeutic agent to the body tissue of a patient, and the method for making such a medical device. More particularly, the invention is directed to a stent having a tubular sidewall with a first coating composition on at least the luminal surface and/or abluminal surface, and a second coating composition disposed on at least a portion of the first coating composition comprising a biodegradable polymer.

### Background Of The Invention

Angioplasty is a procedure that involves placing and inflating a balloon catheter in the blood vessel in the area of blockage, which breaks up the accumulated plaque and opens the vessel. While this technique works well in the short term, many angioplasty procedures require further treatment within six months because of incomplete plaque removal and formation of scar tissue as a result of irritation of the blood vessel known as restenosis. Restenosis results in significant morbidity and mortality and requires further interventions such as repeat angioplasty, coronary bypass, laser surgery or local drug delivery.

Intravascular stenting (the placement of a supporting structure within a blood vessel) has demonstrated success in preventing further interventions in patients. Stents provide structural support to keep the vessel walls from closing and minimize the problem of artertial blockage caused by plaque falling into the vessel after inflation. Intravascular stenting is now commonly involved in 70-90% of procedures performed. Stents can be used as an alternative to or in combination with angioplastly.

Exposure to stents, which are implanted or inserted into the body of a patient, can cause the body tissue to exhibit adverse physiological reactions. For instance, the insertion or implantation of certain stents can lead to the formation of emboli or clots in blood vessels. Other adverse reactions to stents include cell proliferation which can lead to hyperplasia, occlusion of blood vessels, platelet aggregation, and calcification.

In order to address such adverse effects, stents have incorporated therapeutic agents. Such materials can be incorporated into the materials used to make the device. Alternatively, the therapeutic agent(s) can be included in a coating that is applied to a surface of the medical device.

Stents that include a therapeutic agent can be used for direct or local administration of the therapeutic agent to a particular part of the patient's body. For instance, stents having coatings that include a therapeutic agent can be used to prevent restenosis. In some instances, the coating can also include a polymeric material that controls the delivery or release of the therapeutic agent. For example, various types of coated stents in which the coating includes a therapeutic agent have been used for localized delivery of drugs to a body lumen. *See, e.g.,* U.S. Patent No. 6,099,562 to Ding et al.

Such direct or local administration may be more preferred than systemic administration of a therapeutic agent. Systemic administration requires larger amounts and/or higher concentrations of the therapeutic agent because of indirect delivery of such materials to the afflicted area. Also, systemic administration may cause side effects which may not be a problem when the therapeutic agent is locally administered.

Although there are many advantages to incorporating a therapeutic agent into a stent, computational fluid dynamics models for implanted stents coated with a therapeutic agent show that about 1/5 of the total amount of therapeutic agent that is released from the stent is delivered to the blood vessel wall, while about 4/5 of the total amount of therapeutic agent released from the stent is delivered to the blood stream. In particular, these models indicate that a burst release of the therapeutic agent into the bloodstream occurs within 48 hours of implantation or delivery of the stent. In general, the therapeutic agent located on the luminal surface of the stent or stent struts, or located on the side surfaces of the stent struts, is released into the bloodstream during this burst release. This burst release is followed by a lower sustained release of the therapeutic agent.

After implantation of the stent, it is intended that, the stent or struts of the stent should become covered with a new intima layer consisting of endothelial cells. This cell growth occurs generally around the parts of the stent that are exposed to the bloodstream. To minimize excessive neo-intima thickening, which may result from arterial and vascular injuries causing thrombus and inflammatory responses, it might be desirable to deliver therapeutic agents to the intima layer to suppress hyperplasia. However, because a large percentage of the burst release of the therapeutic agent is lost to the bloodstream, less therapeutic agent remains to be released to the neointimal layer during the sustained release period.

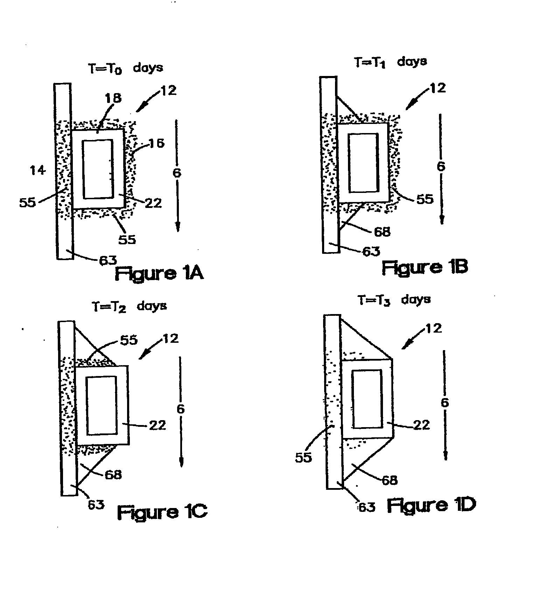

**Figures 1a-1d** depict how the burst release of the therapeutic agent causes it to be lost to the bloodstream so that only a limited amount of the therapeutic agent remains to be released to the intima growth. As shown in **Figure 1a**, when a stent having a strut **12** is implanted in a blood vessel **63**, the therapeutic agent **55** is immediately released from the coating composition **22** applied to the side surfaces **18** and luminal surface **16** of a stent strut **12** to the bloodstream **6**. **Figures 1b-1d**, show that as the neointimal tissue made of endothelial cells **68** grows around the strut, a greater percentage of the therapeutic agent **55** released from the strut at a given time is absorbed into the tissue **68**. However by the time this tissue **68** forms, the release of the therapeutic agent **55** has slowed down dramatically. As a result, the majority of the therapeutic agent **55** released during the first few weeks does not act on the tissue **68**. This results in a low concentration of the therapeutic agent 55 left for delivery to target neo-intima thickening from the endothelial growth, as shown in **Figures 1b-1d****.**

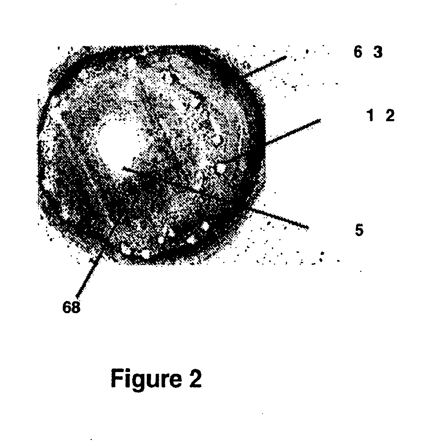

When directly adjacent tissue is not exposed to therapeutic agent or not exposed to sufficient therapeutic agent, occlusion and restenosis can result despite the loading of therapeutic agent onto or into the stent **Figure 2** shows a cross section of a stent 10 having struts 12 implanted in a blood vessel **63**. Within days after implantation, the endothelial cells **68** begin to encapsulate the stent struts. However platelets, fibrin and neutrophils accumulate as well at the stent site. At 14 to 30 days, chronic inflammation may develop and smooth muscle cells can begin to protrude from abluminal to luminal stent areas, causing a hyperplasia effect whereby the intimal layer **5** thickens to the point of occlusion and restenosis as shown in **Figure 2**. Without effective delivery of a therapeutic agent to target undesired cell growth, such cell growth continues to occur radially inward towards the center of the blood vessel.

Also, in some instances it is desirable to deliver a therapeutic agent to certain portions of a body lumen by selectively coating or placing the therapeutic agent on certain parts of the stent or stent struts. For example, it may be desirable to only coat the abluminal surfaces of a stent strut with a therapeutic agent because the preferred endothelial coverage on the luminal site can be reduced by the same therapeutic substance that is targeted to suppress smooth muscle cell growth. However, it may be difficult to only coat one surface of a stent strut with the therapeutic agent because of limitations in the adhesion of the coating containing the therapeutic agent to the strut surface.

In sum, ineffective delivery of a therapeutic agent results in loss of the therapeutic agent to the blood stream, and inadequate control of undesired cell growth. Thus, there is a need for a stent having a therapeutic agent that can remain on or in the stent over a desired period of time to address such undesired cell growth, which develops only weeks after implanting the stent. In particular, there is a need for a stent having a therapeutic agent where the therapeutic agent is not released in a burst but is released at a lower rate so that there is an adequate amount of the therapeutic agent for addressing the undesired cell growth. Also, there is a need to selectively coat stent surfaces with a therapeutic agent.

### Summary of the Invention

These and other objectives are accomplished by the present invention. The present invention is directed to a medical device with a therapeutic agent coating designed to prevent loss of therapeutic agent to the blood stream, to retain therapeutic agent for delivery to cells, to expose all cells to desired therapeutic agent conditions, and modulate therapeutic agent release as desired. The present invention is directed to a stent that comprises a means of delaying the release of a therapeutic agent from certain parts of a medical device. The present invention provides a coating composition and/or layers of coating compositions that reduces or delays the release of a therapeutic agent. Upon deployment of the stent in a blood vessel, the therapeutic agent is initially held back wholly or partly by a release-limiting, biodegradable coating composition. Over time as endothelial cells encapsulate the stent struts and the coating composition is absorbed into the surrounding cell growth, the therapeutic agent is allowed to be released from the surface as governed by mass transport mechanisms and kinetic drug release (KDR) profiles.

In one embodiment, the invention is an intravascular stent designed for implantation into a blood vessel of a patient The stent comprises a tubular stent sidewall structure comprising a plurality of struts and openings in the sidewall structure. At least one strut comprises an abluminal surface, and a luminal surface opposite the abluminal surface. There is a first coating composition comprising a first polymer and a therapeutic agent disposed on the abluminal surface and luminal surface. There is a second coating composition disposed on a portion of the first coating composition that is disposed on the luminal surface. The second coating composition is biodegradable and comprises a bioabsorbable polymer.

In another embodiment, there is disclosed an intravascular stent designed for implantation into a blood vessel of a patient. The stent comprises a tubular stent sidewall structure, comprising a plurality of struts and openings in the sidewall structure. At least one strut comprises an abluminal surface, and a luminal surface opposite the abluminal surface. There is a first side surface and a second side surface opposite the first side surface, in which each side surface is disposed between the abluminal and luminal surfaces. There is a first coating composition comprising a first polymer and a therapeutic agent disposed on the abluminal surface. The first coating composition is not disposed on the luminal or side surfaces. There is a second coating composition disposed on the luminal surface, and on the first side surface and second side surface and on a portion of the first coating composition that is disposed on the abluminal surface. The second coating composition is biodegradable and comprises a bioabsorbable polymer.

In another embodiment, there is an Intravascular stent designed for implantation into a blood vessel of a patient. The stent comprises a tubular stent sidewall structure comprising a plurality of struts and openings in the sidewall structure. At least one strut comprises an abluminal surface, a luminal surface opposite the abtuminal surface. The at least one strut comprise an outer region adjacent to the abtuminal and luminal surfaces, and an inner region adjacent to the outer region. The abluminal surface, luminal surface and outer region comprise a metal or metal oxide having a plurality of pores therein. The inner region is substantially non-porous. A therapeutic agent is disposed in the pore. And there is a coating composition that is biodegradable and comprises a bioabsorbable polymer disposed on at least a portion of the luminal surface. The abluminal surface is free of the coating composition, when the stent is implanted, the abluminal surface is in direct contact with the blood vessel.

In another embodiment, the present invention comprises an intravascular stent designed for implantation into a blood vessel of a patient. The stent comprises a sidewall structure wherein there are a plurality of struts and openings in the sidewall structure. At least one strut comprises an abluminal surface, and a luminal surface opposite the abluminal surface. There is a first coating disposed on the abluminal and luminal *surfaces comprising* a metal or metal oxide having a plurality of pores therein and a therapeutic agent disposed in the pores. There is a second coating disposed on the first coating that is disposed on the luminal surface wherein the second coating is biodegradable and comprises a bioabsorbable polymer. The abluminal surface is free of the second coating composition and when the stent is implanted, the abluminal surface is in direct contact with the blood vessel.

In yet another embodiment, there is a bioabsorbable intravascular stent designed for implantation into a blood vessel of a patient comprising a tubular stent sidewall structure comprising a plurality of struts and openings in the sidewall structure. At least one strut comprises a bioabsorbable material. The strut comprises an abluminal surface, a luminal surface opposite the abluminal surface and a cavity within the strut that is in fluid communication with the abluminal surface. There is a therapeutic agent within the cavity.

In one embodiment, the present invention comprises a method of making an intravascular stent designed for implantation into a blood vessel of a patient. The method comprises a tubular stent sidewall structure comprising a plurality of struts and openings in the sidewall structure. At least one strut comprises an abluminal surface in direct contact with the vessel wall, and a luminal surface opposite the abluminal surface in contact with the blood stream. The method comprises forming a first coating composition comprising a first polymer and a therapeutic agent and disposing the first coating composition on the abluminal surface and luminal surface. The method also comprises forming a second coating composition wherein the second coating composition is biodegradable and comprises a bioabsorbable polymer and disposing the second coating composition on a portion of the first coating composition that is disposed on the luminal surface.

In another embodiment, there is a method of making an intravascular stent designed for implantation into a blood vessel of a patient comprising providing a tubular stent sidewall structure, comprising a plurality of struts and openings in the sidewall structure. At least one strut comprises an abluminal surface, and a luminal surface opposite the abluminal surface. There is a first side surface and a second side surface opposite the first side surface, in which each side surface is disposed between the abluminal and luminal surfaces. The method also comprises forming a first coating composition comprising a fist polymer and a therapeutic agent and disposing the first coating composition on the abluminal surface wherein the first coating composition is not disposed on the luminal or side surfaces. The method comprises forming a second coating composition wherein the second coating composition is biodegradable and comprises a bioabsorbable polymer, and disposing the second coating composition on a portion of the first coating composition that is disposed on the abluminal surface, on the luminal surface, and on the first side surface and second side surface.

In another embodiment, the present invention discloses a method of making an intravascular stent designed for implantation into a blood vessel of a patient comprising: providing a tubular stent sidewall structure, comprising a plurality of struts and openings in the sidewall structure. At least one strut comprises an abluminal surface, and a luminal surface opposite the abluminal surface. There is a first side surface and a second side surface opposite the first side surface, in which each side surface is disposed between the abluminal and luminal surfaces. The method comprises forming a first coating composition comprising a first polymer and a therapeutic agent and disposing the first coating composition on the luminal surface. The first coating composition is not disposed on the abluminal or side surfaces. The method Further comprises forming a second coating composition wherein the second coating composition is biodegradable and comprises a bioabsorbable polymer. The second coating composition is disposed on a portion of the first coating composition that is disposed on the abluminal surface, on the luminal surface, and on the first side surface and second side surface.

In another embodiment, there is a method of making an intravascular stent designed for implantation into a blood vessel of a patient, comprising providing a tubular stent sidewall structure comprising a plurality of struts and openings in the sidewall structure. At least one strut comprises an abluminal surface, a luminal surface opposite the abluminal surface. The at least one strut comprise an outer region adjacent to the abluminal and luminal surfaces, and an inner region adjacent to the outer region. The abluminal surface, luminal surface and outer region comprise a metal or metal oxide have a plurality of pores therein; and the inner region is substantially non-porous, and a therapeutic agent is disposed in the pores. The method also comprises forming a coating composition that is biodegradable and comprises a bioabsorbable polymer and disposing the coating composition on at least a portion of the luminal surface. The abluminal surface is free of the coating composition and when the stent is implanted, the abluminal surface is in direct contact with the blood vessel.

In another embodiment, a method of making an intravascular stent designed for implantation into a blood vessel of a patient comprises providing a plurality of struts and openings in a sidewall structure; wherein at least one strut comprises an abluminal surfaces, and a luminal surface opposite the abluminal surface. The method comprises forming a first coating comprising a metal or metal oxide having a plurality of pores therein, disposing a first coating on the abluminal and luminal surfaces and disposing a therapeutic agent in the pores after the first coating is disposed on the abluminal and luminal surfaces and forming a second coating wherein the second coating is biodegradable and comprises a bioabsorbable polymer. The second coating disposed on the first coating that is disposed on the luminal surface is disposed in a manner so that the abluminal surface is free of the coating composition and that when the stent is implanted, the abluminal surface is in direct contact with the blood vessel.

In another embodiment, a method of making a bioabsorbable intravascular stent designed for implantation into a blood vessel of a patient comprises providing a tubular stent sidewall structure comprising a plurality of struts and openings in the sidewall structure. At least one strut comprises a bioabsorbable material. The strut comprises an abluminal surface, a luminal surface opposite the abluminal surface and a cavity within the strut that is in fluid communication with the abluminal surface. The method further comprises disposing a therapeutic agent within the cavity.

### Brief Description the Drawing

**Figures 1a-1d** show a stent strut with endothelial cell growth and loss of a therapeutic agent to the bloodstream over time.

**Figure 2** is a photograph depicting a perspective view of an implanted intravascular stent where endothelializadon of the stent has occurred to the point of occlusion and restenosis.

**Figure 3** shows an example of an intravascular stent having a middle portion disposed between two end portions.

**Figure 3a** shows the abluminal, luminal, and side surface of a strut of **Figure 3****.**

**Figure 3b** shows a cross-section of a stent with struts including the abluminal, luminal and side surfaces of a strut of Figure 3.

**Figures 4a-4d** depict a strut of a stent, which is described herein, having a coating composition releasing therapeutic agent over time.

**Figures 5a and 5b** depict a strut of a stent, which is described herein, having a second coating composition covering a first coating composition that is disposed on either the abluminal or luminal surfaces of the strut.

**Figure 6a and 6b** demonstrate a strut of a stent in which the strut has a porous surface with a therapeutic agent covered with a coating composition.

**Figures 7a-7c** show a cross section of a strut of a stent, which is described herein, with a reservoir filled with therapeutic agent, in which the struts biodegrade over time.

**Figures 8a-8c** show a cross-section of a strut with a cavity of different shapes, and comprising different volumes within the strut.

### Detailed Description of the Invention

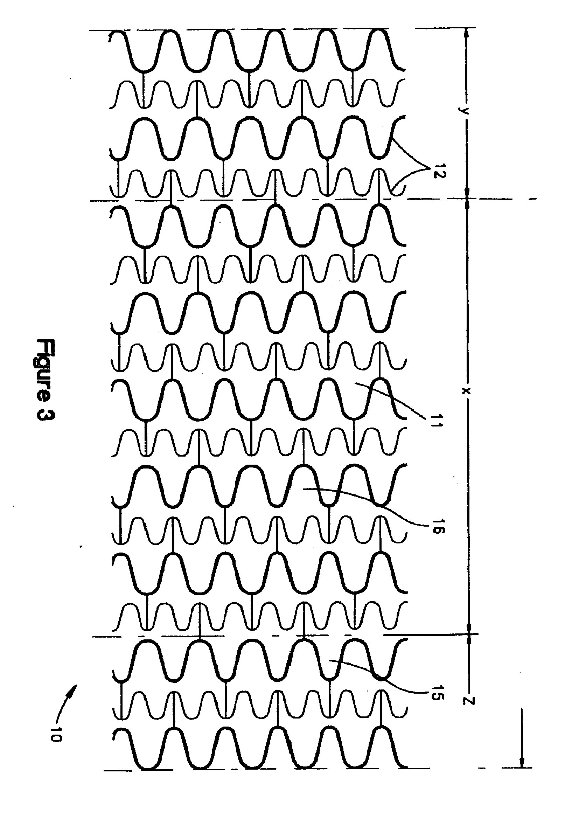

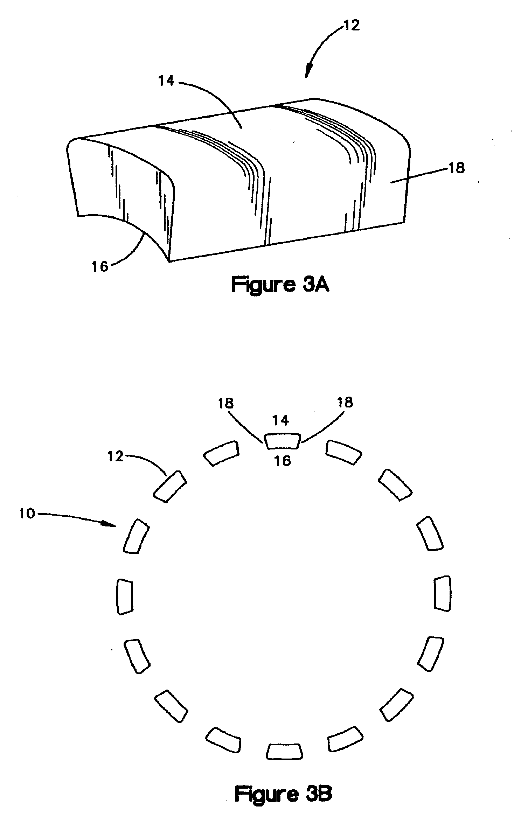

**Figures 3** shows an example of a medical device that is suitable for use in the present invention. This figure shows an implantable intravascular stent **10** comprising a sidewall **11** which comprises a plurality of struts **12** and at least one opening 15 in the sidewall **11**. Generally, the openings **15** are disposed between adjacent struts **12**. This embodiment is an example of a stent where the struts and openings of the stent define a sidewall stent structure having openings therein. Also, the sidewall **11** may have a first sidewall surface 16 and an opposing second sidewall surface, which is not shown in **Figure 3**. The first sidewall surface **16** can be an outer sidewall surface, which faces the body lumen wall when the stent is implanted, or an inner sidewall surface, which faces away from the body lumen wall. Likewise, the second sidewall surface can be an outer sidewall surface or an inner sidewall surface. The stent **10** comprises a middle portion x and two end portions y and z.Generally, the combined end sections comprise about 20% or less of the overall length of the stent.

**Figure 3a** is a cross-sectional view of a stent strut **12** depicted in **Figure 3**. Generally, each individual strut **12** has an outer surface or abluminal surface **14**, an inner surface 16 opposite the outer surface, or luminal surface **14**, and at least one side surface **18**. **Figure 3b** depicts a cross section of a stem 10 with struts. The abluminal surface 14 of the strut **12** is the surface that comes in direct contact with the body lumen wall when the stent is implanted. The abluminal surface **14** need not include only one flat surface or facet. Instead, it can be rounded, such as in the case of a wire strut **12**, or have a number of facets. The luminal surface **16** of the strut **12** is the surface that is opposite the abluminal surface **14**. The two side surfaces **18** are the surfaces of the strut **12** that are adjacent to the luminal surface **16** and abluminal surface **14**. The side surfaces **18** connects the luminal surface **16** and the abluminal surface **14**. Like the abluminal surface **14**, the luminal surface **16** and side surfaces **18** can be rounded or have a number of facets.

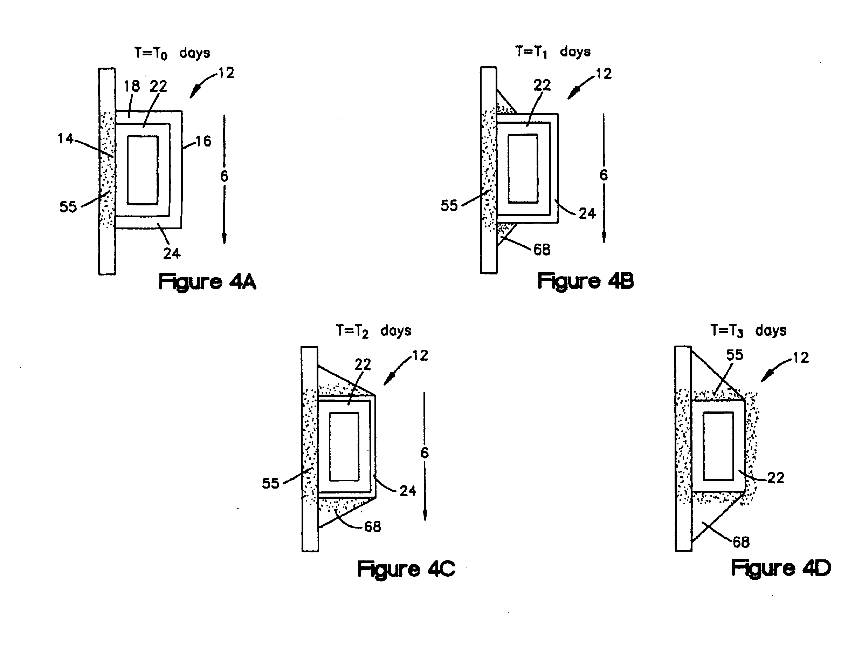

**Figures 4a-4d** depicts one embodiment of the present invention. In this embodiment, a first coating composition **22** is disposed on the abluminal surface **14**, luminal surface **16**, and side surfaces **18** of a stent strut **12**. There is a second coating composition 24 disposed on the portion of the first coating composition **22** that is disposed on the luminal surface **16** and side surfaces **18**. Unlike in **Figures 1a-1d****,** the second coating composition 24 prevents the therapeutic agent 55 from being released too quickly into the bloodstream. As **Figures 4b and 4c** show, over time endothelial cell growth **68** occurs and the second coating composition **24** degrades. In **Figure 4d**, when the second coating composition **24** has degraded, the therapeutic agent **55** can be released to the endothelial cells **68** which surround the stent strut **12**. As a result, there is sufficient therapeutic agent **55** to treat the endothelial cells **68**.

In one embodiment, the first coating composition **22** comprises a first polymer and a therapeutic agent **55**. The first polymer is preferably biostable. In other aspects of the invention, the first coating composition **22** can comprise one or more therapeutic agents. It is also possible that more than one polymer is used to form the first coating composition **22**. The second coating composition **24** comprises a biodegradable or bioabsorbable polymer. It is possible that the second coating composition **24** also contains therapeutic agent **55** or is free of any therapeutic agent when applied to the first coating composition. The polymer of the first coating composition **22** and the second coating composition **24** can be the same or different. In the event both polymers of the first coating composition **22** and the second coating composition **24** are biodegradable or bioresorbable, the first coating composition **22** must degrade at a slower rate than the second coating composition **24**. The therapeutic agents in the first and the second coating composition can be the same or different.

In alternative embodiments not shown, it is possible that first coating composition **22** is only disposed on the abluminal surface **14**, and/or luminal surfaces **16**. It is also possible that the first coating composition **22** is disposed on the abluminal surface **14**, and the side surfaces **18**, or the luminal surfaces **16**, and the side surfaces **18**. The second coating composition can be disposed on at least the portion of the first coating composition **22** that is disposed on the luminal surface **16** and side surfaces **18**; abluminal surface **14** and side surfaces **18**; only the abluminal surface **14**; or only the luminal surface **16**. Further still, the second coating composition can be disposed on the abluminal surface **14**, luminal surface **16**, and the side surfaces **18**.

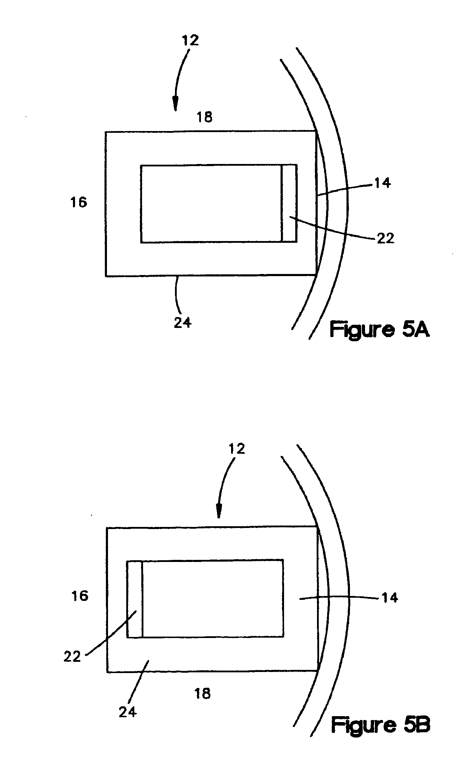

**Figures 5a and 5b** show a cross-sectional view of a strut in additional embodiments of the invention. As show in **Figures 5a**, a first coating composition **22** is disposed on the abluminal surface **14** of the stent strut **12**. The first coating composition is not disposed on the side surfaces **18** or the luminal surface **16**. There is a second coating composition **14** disposed on the portion of the first coating composition **11** that is disposed on the ablumsnal surface **14**, and is also disposed on the luminal surface **16** and side surfaces **18** of the strut **12**. In **Figure 5b**, a first coating composition **22** is disposed on the luminal surface **16** of the stent strut **11**. The first coating composition is not disposed on the side surfaces **18** or the abluminal surface **14**. There is a second coating composition **24** disposed on the portion of the first coating composition **22** that is disposed on the luminal surface **16**, and is also disposed on the abluminal surface **14** and side surfaces **18** of the strut **12**.

In the above embodiments of **Figures 5a and 5b**, the first coating composition **22** can comprise a first polymer and a therapeutic agent. The first polymer is preferably biostable. In other aspects of the invention, the first coating composition can comprise one or more therapeutic agents. It is also possible that more than one polymer is used to create the first coating composition. The second coating composition comprises a biodegradable and/or bioabsorbable polymer. It is possible the second coating composition also contains therapeutic agent or is free of any therapeutic agent when applied to the first coating composition. The polymer of the first coating composition and the second coating composition can be the same or different. The therapeutic agents in the first and the second coating composition can be the same.

In the embodiments shown in **Figures 5a and 5b****,** the second coating composition **24** can help retain the first coating composition on the abluminal or luminal sides of the strut In particular, the second coating composition **24** encapsulates the stent strut **12** and helps to secure the first coating composition **22** on the abluminal surface **14** or luminal surface 16 of the strut 12 since the first coating composition **22** may have limited adhesion to the abluminal or luminal surface. In a preferred embodiment the second coating composition **24** comprises a biostable, biodegradable sad/or bioabsorbable polymer. When the second coating, composition **24** degrades, the surfaces of the strut **12** that are not coated with the second coating composition **24** or first coating composition **22** will be bare.

It is also feasible that the first coating composition **22** is disposed on the abluminal surface **14**, and the side surfaces **18**, or the luminal surfaces **16**, and the side surfaces **18**. In another embodiment not shown, the first coating composition **22**, can be disposed on both the abluminal surface **14** and luminal surface **16**. The first coating composition **22** can also be disposed on the abluminal surface **14**, luminal surface **16** and on the side surfaces **18**. The second coating composition **24** can be disposed on at least the portion of the first coating composition **22** that is disposed on the luminal surface **16** or abluminal surface **14** and side surfaces **18**. Further still, the second coating composition can be disposed on the abluminal surface **14**, luminal surface 16, and the side surfaces **18**.

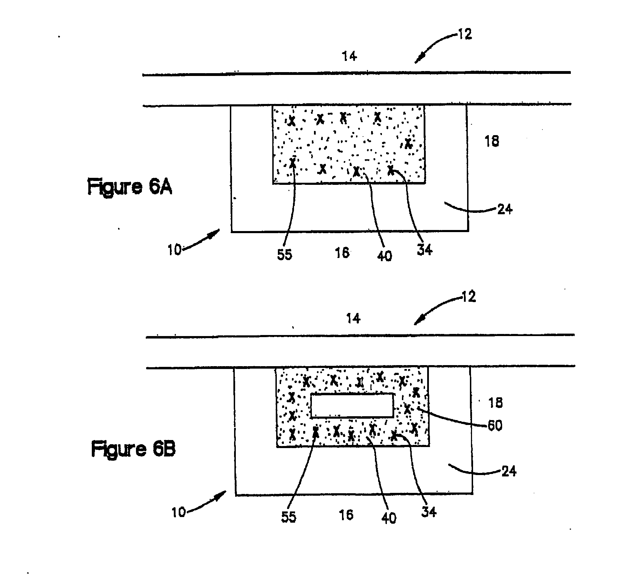

**Figures 6a and 6b** depict another embodiment of the invention. In Figure **6a****,** the stent strut comprises a plurality of pores **40** present in the abluminal surface **14**, luminal surface **16**, and side surfaces **18**. In particular, the stent strut **12** includes an inner portion **54** and a porous outer portion **34**, which includes the surfaces of the stent strut **12**. A therapeutic agent is disposed in the pores of the porous outer portion **34**. There is also an outer coating composition **24** disposed on the luminal surface **16**, and side surfaces **18** to delay or reduce the release of the therapeutic agent from the pores. Alternatively, the outer coating composition **24** may be disposed only on the luminal surface.

**In** **Figure 6b**, there is an inner coating composition **60** disposed on the abluminal surface **14**, luminal surface **16**, and side surfaces **18** of the strut. There is also an outer coating composition **24,** disposed on the inner coating composition **60** disposed on the luminal surface **16**, and side surfaces **18** to delay or reduce the release of the therapeutic agent from the pores. Alternatively, the outer coating composition **24** may be disposed only on the inner coating composition **60** disposed on the luminal surface. The inner coating composition **60** can comprise a polymer with a plurality of pores **40**. In alternative embodiments, the inner coating composition **60** can comprise a metal oxide or metal having a plurality of pores **40** therein. In still other embodiments, the inner coating composition **60** can comprise any material that is capable of forming a porous surface.

In the embodiments of **Figures 6a and 6b**, the plurality of pores **40** can be present in the abluminal surface **14** only, or on the luminal surface **16** only. Alternatively, the plurality of pores **40** can be in the abluminal surface **14** and side surfaces **18** or the luminal surface **16** and the side surfaces **18**. Some of the pores **40** can be interconnected. At least some of the pores **40** may contain therapeutic agent. It is possible that the pores **40** may contain more than one type of therapeutic agent. The therapeutic agent **55** can partially or entirely fill a pore **40.** The pores can also serve as an exit port for therapeutic agent by being connected to a larger reservoir containing therapeutic agent.

Also, in the embodiments, the outer coating composition **24** can comprise one or more polymers. The outer coating composition **24** preferably comprises a bioabsorbable or biodegradable polymer. The outer coating composition **24** can be disposed on any portion of the porous surface of the stent **10** or inner coating composition **60**. The inclusion allows the strut to be endothelialized before allowing a significant amount of the therapeutic material to be released from the pores.

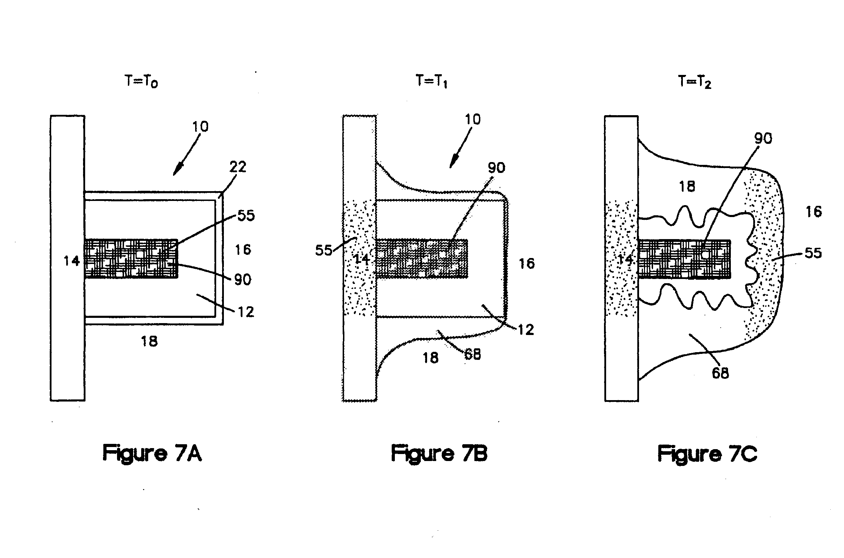

**Figures 7a-7c** shows a cross-sectional view of another embodiment in which a strut has a cavity **90** that contains a therapeutic agent **55.** In some embodiments the cavity **90** can also include a polymer. **Figure 7a** shows an embodiment of a stent strut **12** having a cavity **90** that extends to the abluminal surface **14** of the strut **12.** In this embodiment, the cavity **90** does not extend to the luminal surface **16** of the strut **12**. In other embodiments, the cavity **90** can extend to the luminal surface **16** of the strut **12**. In the embodiment shown in Figure **7a****,** there is a first coating composition **22** disposed on luminal surface **16**, and side surfaces **18** of the strut **12**. The stent strut **12** is biodegradable. Preferably, the first coating composition **22** comprises a biodegradable polymer. The first coating composition **22** can comprise more than one polymer, some of which degrade at faster rates than others. Alternately, the first coating composition **22** can comprise layers of biodegradable polymer(s). In other embodiments, the first coating composition **22** can be disposed on the abluminal surface **14** and/or the luminal surface **16.** It is also possible the first coating composition **22** is disposed on the abluminal surface **14** and the side surfaces **18**, or alternatively on the luminal surface 16 and the side surfaces 18. Although not shown in the figure, there may also be a second coating composition comprising a biodegradable polymer. In alternative embodiments, the second coating composition can also comprise one or more therapeutic agents for immediate release. The first coating composition **22** degrades as shown in **Figure 7b** after implantation of the stent When the first coating composition **22** that was disposed on the abluminal surface **14** of the strut **12** degrades, the therapeutic agent **55** in the cavity **90** is released to the vessel wall. Preferably, the first coating composition **22** degrades at a rate allowing the therapeutic agent to be released between about 1 hour to about 14 days.

As shown in **Figure 7c****,** over time the stent strut **12** degrades and allows the therapeutic agent **55** in the cavity **90** to be released to the luminal side **16** of the strut **12** or to the tissue **68** that surrounds the luminal surface **16** of the strut **12**. Therefore, this embodiment delays release of the therapeutic agent **55** to the luminal surface **16** of the stent strut **12** until after endothelializstion has occurred.

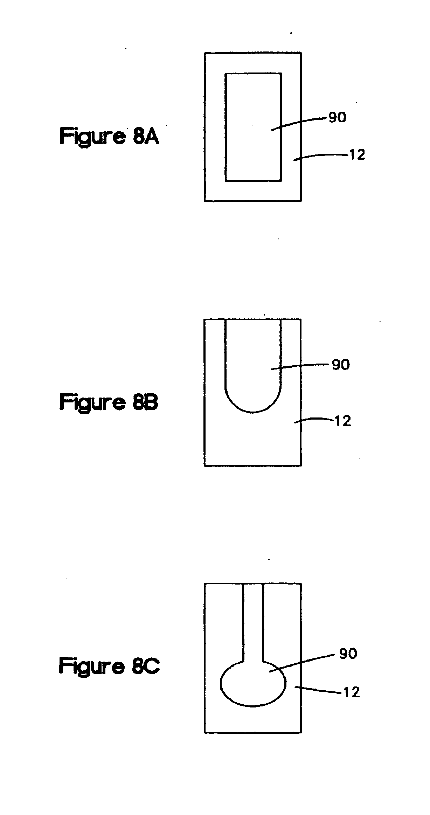

**Figures 8a, 8b,** **and 8e** show a cross-sectional view of stent struts having cavities that can be a variety of shapes and sizes. In **Figure 8a****,** the cavity **90** can take up a majority of the volume of the strut **12,** and have a square cross-section. Alternatively, the cavity can have a different cross-sectional shape, e.g. triangular, circular, etc. This embodiment would likely maximize the amount of therapeutic agent(s) that can affect a tissue. In **Figure 8b**, the cavity **90** can be shaped in a U-shaped cross-section, which comprises less volume than **Figure 8a**. Lastly, in **Figure 8c****,** the cavity **90** is a bottle-shaped cross-sectional. This further delays the release of therapeutic agent to certain areas, as only smaller amounts will be released from the neck of the cavity **90** relative to the bottom. The cavity may comprise one or more therapeutic agents, and may further comprise polymers as necessary to delay release of therapeutic agent. In each case, a desired timely distribution and possibly also a spatial distribution of the released amount of therapeutic agent may be set by the shape or dimensioning of the cavities.

### A. Medical Devices

Medical devices that are particularly suitable for the present invention include any kind of stent for medical purposes which is known to the skilled artisan. Preferably, the stents are intravascular stents that are designed for permanent implantation in a blood vessel of a patient and that have an sidewall stent structure with openings. Suitable intravascular stents include self expanding stents and balloon expandable stents. Examples of self expanding stents useful in the present invention are illustrated in U.S. Patent Nos. 4,655,771 and 4,954,126 issued to Wallsten and 5,061,275 issued to Wallsten et aL Examples of appropriate balloon expandable stents are shown in U.S. Patent No. 5,449,373 issued to Pinchasik et al. In certain embodiments, the stent comprises a sidewall stent structure with openings. When such stents are used, it is in some instances preferable to have the coating disposed on the stent to conform to the stent to preserve the openings of the sidewall structure. In preferred embodiments, the stent suitable for the present invention is an Express stent. More preferably, the Express stent is an Express™ stent or an Express2™ stent (Boston Scientific, Inc. Natick, Mass.).

Stents that are suitable for the present invention may be fabricated from metallic, ceramic, polymers, or a combination thereof. Preferably, the materials are biocompatible. Metallic material is more preferable. Suitable metallic materials include metals and alloys based on titanium (such as nitinol, nickel titanium alloys, thermo memory alloy materials), stainless steel, niobium, tantalum, nickel chrome, or certain cobalt alloys including cobalt chromium nickel alloys such as Elgiloy® and Phynox®. Metallic materials also include clad composite filaments, such as those disclosed in WO 94/16646.

Suitable ceramic materials include, but are not limited to, oxides, carbides, or nitrides of the transition elements such as titanium oxides, hafnium oxides, iridiumoxides, chromium oxides, aluminum oxides, and zirconiumoxides. Silicon based materials, such as silica, may also be used. The polymer may be biostable. Also, the polymer may be biodegradable. Suitable polymers include, but are not limited to, styrene isobutylene styrene, polyetheroxides, polyvinyl alcohol, polyglycolic acid, polylactic acid, polyamides, poly-2-hydroxy-butyrate, polycaprolactone, poly(lactic-co-clycolic)acid, and Teflon.

Polymers may be used for forming the stent in the present invention include without limitation isobutylene-based polymers, polystyrene-based polymers, polyacrylates, and polyacrylate derivatives, vinyl acetate-based polymers and its copolymers, polyurethane and its copolymers, silicone and its copolymers, ethylene vinyl-acetate, polyethylene terephtalate, thermoplastic elastomers, polyvinyl chloride, polyolefins, cellulosics, polyamides, polyesters, polysulfones, polytetrafluorethylenes, polycarbonates, acrylonitrile butadiene styrene copolymers, acrylics, polylactic acid, polyglycolic acid, polycaprolactone, polylactic acid-polyethylene oxide copolymers, cellulose, collagens, and chitins.

Other polymers that are useful as materials for stents include without limitation dacron polyester, poly(ethylene terephthalate), polycarbonate, polymethylmethacrylate, polypropylene, polyalkylene oxalates, polyvinylchloride, polyurethanes, polysiloxanes, nylons, poly(dimethyl siloxane), polycyanoacrylates. polyphosphazenes, poly(amino acids), ethylene glycol I dimetbacrylate, poly(methyl methacrylate), poly(2-hydroxyethyl methacrylate), polytetrafluoroethylene poly(HEMA), polyhydroxyalkanoates, polytetrafluorethylene, polycarbonate, poly(glycolide-lactide) copolymer, polylactic acid, poly(γ-caprolactone), poly(γ -hydroxybutyrate), polydioxanone, poly(γ -ethyl glutamate), polyiminocarbonates, poly(ortho ester), polyanhydrides, alginate, dextran, chitin, cotton, polyglycolic acid, polyurethane, or derivatized versions thereof, i.e., polymers which have been modified to include, for example, attachment sites or cross-linking groups, *e.g.,* RGD, in which the polymers retain their structural integrity while allowing for attachment of cells and molecules, such as proteins, nucleic acids, and the like.

Stents may also be made with non-polymers. Examples of useful non-polymers include sterols such as cholesterol, stigmasterol, *β*-sitosterol, and estradiol; cholesteryl esters such as cholesteryl stearate; C₁₂ -C₂₄ fatty acids such as lauric acid, myristic acid, palmitic acid, stearic acid, arachidic acid, behenic acid, and lignoceric acid; C₁₈-C₃₆ mono-, di- and triacylglycerides such as glyceryl monooleate, glyceryl monolinoleate, glyceryl monolaurate, glyceryl monodocosanoate, glyceryl monomyristate, glyceryl monodicenoate, glyceryl dipalmitate, glyceryl didorosanoate, glyceryl dimyristate, glyceryl didecenoate, glyceryl tridocosanoate, glyceryl trimyristate, glyceryl tridecenoate, glycerol tristearate and mixtures thereof; sucrose fatty acid esters such as sucrose distearate and sucrose palmitate; sorbitan fatty acid esters such as sorbitan monostearate, sorbitan monopalmitate and sorbitan tristearate; C₁₆ -C₁₈ fatty alcohols such as cetyl alcohol, myristyl alcohol, stearyl alcohol, and cetostearyl alcohol; esters of fatty alcohols and fatty acids such as cetyl palmitate and ceteatyl palmitate; anhydrides of fatty acids such as stearic anhydride; phospholipids including phosphatidylcholine (lecithin), phosphatidylserine, phosphatidylethanolamine, phosphatidylinositol, and lysoderivatives thereof; sphingosine and derivatives thereof; sphingomyelins such as stearyl, palmitoyl, and tricosanyl sphingomyelins; ceramides such as stearyl and palmitoyl ceramides; glycosphingolipids; lanolin and lanolin alcohols; and combinations and mixtures thereof. Preferred non-polymers include cholesterol, glyceryl monostearate, glycerol tristearate, stearic acid, stearic anhydride, glyceryl monooleate, glyceryl monolinoleate, and acetylated monoglycerides.

The stent of the present invention is made of biodegradable materials that are also biocompatible. Biodegradable means that a material will undergo breakdown or decomposition into harmless compounds as part of a normal biological process. In preferred embodiments, the invention comprises using bioabsorbable polymers. The bioabsorbable polymer can comprise polyelectrolyte components such as heparin, albumin, and gelatin. In one embodiment, the bioabsorbable polymer degrades at a rate of about 95% weight loss per 30 days to about 95% weight loss per hour. The stent may have a plurality of apertures in the body of the to stent promote the successful biodegradation of the stent. Suitable biodegradable materials for the stent include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Biodegradable glass or bioactive glass is also a suitable biodegradable material for use in the present invention. Preferably the materials have been approved by the Food and Drug Adminstration.

### B. Suitable Therapeutic Agents

The term "therapeutic agent" encompasses biologically active material, and also genetic materials and biological materials. The therapeutic agents named herein include their analogs and derivatives. Non-limiting examples of suitable therapeutic agent include heparin, heparin derivatives, urokinase, dextrophenylalanine proline arginine chloromethylketone (PPack), enoxaprin, aagiopeptin, hirudin, acetylsalicylic acid, tacrolimus, everolimus, rapamycin (sirolimus), pimecrolimus, amlodipine, doxazosin, glucocorticoids, betamethasone, dexamethasone, prednisolone, corticosterone, budesonide, sulfasalazine, rosiglitazone, mycophenolic acid, mesalamine, paclitaxel, 5-fluorouracil, cisplatin, vinblastine, vincristine, epothilones, methotrexate, azathioprine, adriamycin, mutamycin, endostatin, angiostatin, thymidine kinase inhibitors, cladribine, lidocaine, bupivacaine, ropivacaine, D-Phe-Pro-Arg chloromethyl ketone, platelet receptor antagonists, anti-thrombin antibodies, anti-platelet receptor antibodies, aspirin, dipyridamole, protamine, hirudin, prostaglandin inhibitors, platelet inhibitors, trapidil, liprostin, tick antiplatelet peptides, 5-azacytidine, vascular endothelial growth factors, growth factor receptors, transcriptional activators, translational promoters, antiproliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin, cholesterol lowering agents, vasodilating agents, agents which interfere with endogenous vasoactive mechanisms, radiochemical, antioxidants, probucol, antibiotic agents, penicillin, cefoxitin, oxacillin, tobranycin, anti-angiogenic agents, fibroblast growth factors, estrogen, estradiol (E2), estriol (E3), 17-beta estradiol, digoxin, beta blockers, captopril, enalopril, statins, steroids, vitamins, paclitaxel (as well as its derivatives, analogs or paclitaxel bound to proteins, e.g. Abraxanon™) 2'-succinyl-taxol. 2'-succinyl-taxol triethanolamine, 2'-glutaryl-taxol, 2'-glutaryl-taxol triethanolamine salt, 2'-O-ester with N-(dimethytamiaoethyl) glutamine, 2'-O-ester with N-(dimethylaminoethyl) glutamide hydrochloride salt, nitroglycerin, nitrous oxides, nitric oxides, antibiotics, aspirins, digitalis, estrogen, estradiol and glycosides. In one embodiment, the therapeutic agent is a smooth muscle cell inhibitor or antibiotic. In a preferred embodiment, the therapeutic agent is taxol (*e*.*g*., Taxol®), or its analogs or derivatives. In another preferred embodiment, the therapeutic agent is paclitexel, or its analogs or derivatives. In yet another preferred embodiment, the therapeutic agent is an antibiotic such as erythromycin, amphotericin, rapamycin, adriamycin, etc.

The term "genetic materials" means DNA or RNA, including, without limitation, of DNA/RNA encoding a useful protein stated below, intended to be inserted into a human body including viral vectors and non-viral vectors.

The term "biological materials" include cells, yeasts, bacteria, proteins, peptides, cytokines and hormones. Examples for peptides and proteins include vascular endothelial growth factor **(VEGF),** transforming growth factor (TGF), fibroblast growth factor (FGF), epidermal growth factor (EGF), cartilage growth factor (CGF), nerve growth factor (NGF), keratinocyte growth factor (KGF), skeletal growth factor (SGF), osteoblast-derived growth factor (BDGF), hepatocyte growth factor (HGF), insulin-like growth factor (IGF), cytokine growth factors (CGF), platelet-derived growth factor (PDGF), hypoxia inducible factor-1 (HIF-1), stem cell derived factor (SDF), stem cell factor (SCF), endothelial cell growth supplement (ECGS), granulocyte macrophage colony stimulating factor (GM-CSF), growth differentiation factor (GDF), integrin modulating factor (IMF), calmodulin (CaM), thymidine kinase (TK), tumor necrosis factor (TNF), growth hormone (GH), bone morphogenic protein (BMP) *(e.g.,* BMP-2, BMP-3, BMP-4, BMP-5, BMP-6 (Vgr-1), BMP-7 (PO-1), BMP-8, BMP-9, BMP-10, BMP-11, BMP-12, HMP-14, BMP-15, BMP-16, etc.), matrix metalloproteinase (MMP), tissue inhibitor of matrix metalloproteinase (TIMP). cytokines, interleukin (*e*.*g*., IL-1, IL-2, IL-3, IL-4, IL-5, IL-6, IL-7, IL-8, IL-9, IL-10, IL-11, IL-12, IL-15, etc.), lymphokines, interferon, integrin, collagen (all types), elastin, fibrillins, fibronectin, vitronectin, lamimn, glycosaminoglycans, proteoglycans, transferrin, cytotactin, cell binding domains (*e*.*g*., RGD), and tenascin. Currently preferred BMP's are BMP-2. BMP-3, BMP-4, BMP-5, BMP-6, BMP-7. These dimeric proteins can be provided as homodimers, heterodimers, or combinations thereof, alone or together with other molecules. Cells can be of human origin (autologous or allogeneic) or from an animal source (xenogeneic), genetically engineered, if desired, to deliver proteins of interest at the transplant site. The delivery media can be formulated as needed to maintain cell function and viability. Cells include progenitor cells (*e*.*g*., endothelial progenitor cells), stem cells (*e*.*g*., mesenchymal, hematopoietic, neuronal), stromal cells, parenchymal cells, undifterentiated cells, fibroblasts, macrophage, and satellite cells.

Other non-genetic therapeutic agents include:
- anti-thrombogenic agents such as heparin, heparin derivatives, urokinase, and PPack (dextrophenylalanine proline arginine chloromethylketone);
- anti-proliferative agents such as enoxaprin, angiopeptin, or monoclonal antibodies capable of blocking smooth muscle cell proliferation, hirudin, acetylsalicylic acid, tacrolimus, everolimus, amlodipine and doxazosin;
- anti-inflammatory agents such as glucocorticoids, betamethasone, dexamethasone, prednisolone, corticosterone, budesonide, estrogen, sulfasalazine, rosiglitazone, mycophenolic acid and mesalamine;
- anti-neoplastic/anti-proliferativelanti-miotic agents such as paclitaxel, 5-fluorouracil, cisplatin, vinblastine, vincristine, epothilones, methotrexate, azathioprine, adriamycin and mutamycin; endostatin, angiostatin and thymidine kinase inhibitors, cladribine, taxol and its analogs or derivatives;
- anesthetic agents such as lidocaine, bupivacaine, and ropivacaine;
- anti-coagulants such as D-Phe-Pro-Arg chloromethyl ketone, an RGD peptide-containing compound, heparin, antithrombin compounds, platelet receptor antagonists, anti-thrombin antibodies, anti-platelet receptor antibodies, aspirin (aspirin is also classified as an analgesic, antipyretic and anti-inflammatory drug), dipyridamole, protamine, hirudin, prostaglandin inhibitors, platelet inhibitors, antiplatelet agents such as trapidil or liprostin and tick antiplatelet peptides;
- DNA demethylating drugs such as 5-azacytidine, which is also categorized as a RNA or DNA metabolite that inhibit cell growth and induce apoptosis in certain cancer cells;
- vascular cell growth promoters such as growth factors, vascular endothelial growth factors (VEGF, all types including VEGF-2), growth factor receptors, transcriptional activators, and translational promoters;
- vascular cell growth inhibitors such as anti-proliferative agents, growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin;
- cholesterol-lowering agents, vasodilating agents, and agents which interfere with endogenous vasoactive mechanisms;
- anti-oxidants, such as probucol;
- antibiotic agents, such as penicillin, cefoxitin, oxacillin, tobranycin, rapamycin (sirolimus):
- angiogenic substances, such as acidic and basic fibroblast growth factors, estrogen including estradiol (E2), estriol (E3) and 17-beta estradiol;
- drugs for heart failure, such as digoxin, beta-blockers, angiotensin-converting enzyme (ACE) inhibitors including captopril and enalopril, statins and related compounds; and
- macrolide agents such as sirolimus, pimecrolimus, or tacrolimus, zotarolimus, everolimus.

Preferred biological materials include anti-proliferative drugs such as steroids, vitamins, and restenosis-inhibiting agents. Preferred restenosis-inhibiting agents include microtubule stabilizing agents such as Taxol®, paclitaxel (*i*.*e*., paclitaxel, paclitaxel analogs, or paclitaxel derivatives, and mixtures thereof). For example, derivatives suitable for use in the present invention include 2'-succinyl-taxol, 2'-succinyl-taxol triethanolamine, 2'-glutaryl-taxol, 2'-glutaryl-taxol triethanolamine salt, 2'-O-ester with N-(dimethylaminoethyl) glutamine, and 2'-O-ester with N-(dimethylaminoethyl) glutamide hydrochloride salt

Other suitable therapeutic agents include tacrolimus; halofuginone; inhibitors of HSP90 heat shock proteins such as geldanamycin; microtubule stabilizing agents such as epothilone D; phosphodiesterase inhibitors such as cliostazole; Barket inhibitors; phospholamban inhibitors; and Serca 2 gene/proteins.

Other preferred therapeutic agents include nitroglycerin, nitrous oxides, nitric oxides, aspirins, digitalis, estrogen derivatives such as estradiol and glycosides.

In one embodiment, the therapeutic agent is capable of altering the cellular metabolism or inhibiting a cell activity, such as protein synthesis, DNA synthesis, spindle fiber formation, cellular proliferation, cell migration, microtubule formation, microfilament formation, extracellular matrix synthesis, extracellular matrix secretion, or increase in cell volume. In another embodiment, the therapeutic agent is capable of inhibiting cell proliferation and/or migration.

In certain embodiments, the therapeutic agents for use in the medical devices of the present invention can be synthesized by methods well known to one skilled in the art. Alternatively, the therapeutic agents can be purchased from chemical and pharmaceutical companies.

Methods suitable for applying therapeutic agents to the devices of the present invention preferably do not alter or adversely impact the therapeutic properties of the therapeutic agent.

### C. Suitable Polymers

Polymers useful for forming the coating compositions should be ones that are biocompatible, particularly during insertion or implantation of the device into the body and avoids irritation to body tissue. Examples of such polymers include, but not limited to, polyurethanes, polyisobutylene and its copolymers, silicones, and polyesters. Other suitable polymers include polyolefins, polyisobutylene, ethylene-alphaolefin copolymers, acrylic polymers and copolymers, vinyl halide polymers and copolymers such as polyvinyl chloride, polyvinyl ethers such as polyvinyl methyl ether, polyvinylidene halides such as polyvinylidene fluoride and polyvinylidene chloride, polyacrylonitrile, polyvinyl ketones, polyvinyl aromatics such as polystyrene, polyvinyl esters such as polyvinyl acetate; copolymers of vinyl monomers, copolymers of vinyl monomers and olefins such as ethylene-methyl methacrylate copolymers, acrylonitrile-styrene copolymers, ABS resins, ethylene-vinyl acetate copolymers, polyamides such as Nylon 66 and polycaprolactone, alkyd resins, polycarbonates, polyoxyethylenes, polyimides, polyethers, epoxy resins, polyurethanes, rayon-triacetate, cellulose, cellulose acetate, cellulose butyrate, cellulose acetate butyrate, cellophane, cellulose nitrate, cellulose propionate, cellulose ethers, carboxymethyl cellulose, collagens, chitins, polylactic acid, polyglycolic acid, and polylactic acid-polyethylene oxide copolymers. Since the polymer is being applied to a part of the medical device which undergoes mechanical challenges, e.g. expansion and contraction, the polymers are preferably selected from elastomeric polymers such as silicones (*e*.*g*. polysiloxanes and substituted polysiloxanes), polyurethanes, thermoplastic elastomers, ethylene vinyl acetate copolymers, polyolefin elastomers, and EPDM rubbers. The polymer is selected to allow the coating to better adhere to the surface of the strut when the stent is subjected to forces or stress. Furthermore, although the coating can be formed by using a single type of polymer, various combinations of polymers can be employed.

Generally, when a hydrophilic therapeutic agent is used then a hydrophilic polymer having a greater affinity for the therapeutic agent than another material that is less hydrophilic is preferred. When a hydrophobictberapeutic agent is used then a hydrophobic polymer having a greater affinity for the therapeutic agent is preferred.

Examples of suitable hydrophobic polymers or monomers include, but not limited to, polyolefins, such as polyethylene, polypropylene, poly(l-butene), poly(2-butene), poly(l-pentene), poly(2-pentene), poly(3-methyl-l-pentem), poly(4-methyl-1-pentene), poly(isoprene), poly(4-methyl- I -pentene), ethylene-propylene copolymers, ethylene-propylene-hexadiene copolymers, ethylene-vinyl acetate copolymers, blends of two or more polyolefins and random and block copolymers prepared from two or more different unsaturated monomers; styrene polymers, such as poly(styrene), poly(2-methylstyrene), styrene-acrylonitrile copolymers having less than about 20 mole-percent acrylonitrile, and styrene-2,2,3,3,-tetrafluoropropyl methacrylate copolymers; halogenated hydrocarbon polymers, such as poly(chlorotrifluoroethylene), chlorotrifluoroethylene-tetrafluoroethylene copolymers, poly(hexafluoropropylene), poly(tetrafluoroethylene), tetragfluoroethylene,_{:} tetrafluoroethylene-ethylene copolymers, poly(trifluoroethylene), poly(vinyl fluoride), and poly(vinylidene fluoride); vinyl polymers, such as poly(vinyl butyrate), poly(vinyl decanoate), poly(vinyl dodecanoate), poly(vinyl hexadecanoate), poly(vinyl hexanoate), poly(vinyl propionate), poly(vinyl octanoate), poly(heptafluoroisopropoxyethylene), poly(heptafluoroisopropoxypropylene), and poly(methacrylonitrile); acrylic polymers, such as poly(n-butyl acetate), poly(ethyl acrylate), poly(1-chlorodifluoromethyl)tetrafluoroethyl acrylate, poly di(chlorofluoromethyl)fluoromethyl acrylate, poly(1,1-dihydrohcptafluorobutyl acrylate), poly(1,1-dihydropentat1uol'Oisopropyl acrylate), poly(1,1-dihydropentadecafluorooctyl acrylate), poly(heptafluoroisopropyl acrylate), poly 5-(heptafluoroisopropoxy)pentyl acrylate, poly 11-(heptafluoroisopropoxy)undecyl acrylate, poly 2-(heptafluoropropoxy)ethyl acrylate, and poly(nonafluomisobutyl acrylate); methacrylic polymers, such as poly(benzyl methacrylate), poly(n-butyl methacrylate), poly(isobutyl methacrylate), poly(t-butyl methacrylate), poly(t-butylaminoethyl methacrylate), poly(dodecyl methacrylate), poly(ethyl methacrylate), poly(2-ethylhexyl methacrylate), poly(n-hexyl methacrylate), poly(phenyl methacrylate), poly(n-propyl methacrylate), poly(octadecyl methacrylate), poly(1,1-dihydropentadecafluorooctyl methacrylate), poly(heptafluoroisopropyl methacrylate), poty(heptadecafluorooctyl methacrylate), poly(l-hydrotetrafluoroethyl methacrylate), poly(1,1-dihydrotetrafluoropropyl methacrylate), poly(l-hydruhexai!uoroisopropyl methacrylate), and poly(t-nonaflwrobutyl methacrylate); polyesters, such a poly(ethylene terephthalate) and poly(butylene terephthalate); condensation type polymers such as and polyurethanes and silvxane-urethane copolymers; polyorganosiloxanes, *i.e.,* polymeric materials characterized by repeating siloxane groups, represented by Ra SiO 4-a/2, where R is a monovalent substituted or unsubstituted hydrocarbon radical and the value of a is 1 or 2; and naturally occurring hydrophobic polymers such as rubber.

Examples of suitable hydrophilic polymers or monomers include, but not limited to; (meth)acrylic acid, or alkaline metal or ammonium salts thereof; . (meth)acrylamide; (metb)acrylonitrile; those polymers to which unsaturated dibasic, such as maleic acid and fumaric acid or half esters of these unsaturated dibasic acids, or alkaline metal or ammonium salts of these dibasic adds or half esters, is added; those polymers to which unsaturated sulfonic, such as 2-acrylamido-2-methylpropanesulfonic, 2-(meth)acryloylethanesulfonic acid, or alkaline metal or ammonium salts thereon is added; and 2-hydroxyethyl (meth)acrylate and 2-hydroxypropyl (meth)acrylate.

Polyvinyl alcohol is also an example of hydrophilic polymer. Polyvinyl alcohol may contain a plurality of hydrophilic groups such as hydroxyl, amido, carboxyl, amino, ammonium or sulfonyl (-SO3). Hydrophilic polymers also include, but are not limited to, starch, polysaccharides and related cellulosic polymers; cylone glycols and oxides such as the polyethylene oxides; polymerized ethylenically unsaturated carboxylic acids such as acrylic, mathacrylic and maleic acids and partial esters derived from these acids and polyhydric alcohols such as the alkylene glycols; homopolymers and copolymers derived from acrylamide; and homopolymers and copolymers of vinylpyrrolidone.

Preferably, for stents which undergo mechanical challenges, *e.g.,* expansion and contraction, polymers should be selected from elastomeric polymers such as silicones (e.g., polysiloxanes and substituted polysiloxanes), polymethanes, thermoplastic elastomers, ethylene vinyl acetate copolymers, polyolefin elastomers, and EPDM rubbers. Because of the elastic nature of these polymers, the coating composition is capable of undergoing deformation under the yield point when the device is subjected to forces, stress or mechanical challenge.

### D. Methods for Making the Coatings

The coating compositions suitable for the device described herein can be prepared by dissolving or suspending a polymer and/or therapeutic agent in a solvent. Solvents that may be used to prepare coating compositions include ones which can dissolve or suspend the polymer and/or therapeutic agent in solution. Examples of suitable solvents include, but are not limited to, water, such as hot water, tetrahydrofuran, methylethylketone, chloroform, toluene, acetone, isooctane, 1,1,1, trichloroethane, dichloromethane, isopropanol, IPA, and mixture thereof.

The aforementioned coated medical devices can be made by applying coating compositions onto the surface of the medical device. Coating compositions can be applied by any method to a surface of a medical device or to another coating composition known by one skilled in the art. The different surfaces may be coated by the same or different methods. Suitable methods for applying the coating compositions to the medical devices include, but are not limited to, spray-coating, painting, rolling, electrostatic deposition, ink jet coating, and a batch process such as air suspension, pan-coating or ultrasonic mist spraying, or a combination thereon

In embodiments where a coating composition is to be applied to fewer than all the surfaces of the struts of a stent, such as on the stets described above in **Figure 4a**, **Flgure 5a** and **Figure 5b** it is preferable to employ coating methods that selectively apply the coating composition. For instance, a first or second coating composition can be deposited onto a substrate. The substrate is preferably made from materials that has minimal adhesion to the coating composition so that the coating composition can be easily removed and transferred to the surface. For instance, in the embodiment of **Figure 4a**, the first coating composition **22** can be applied to the abluminal surface **14,** luminal surface **16** and side surfaces **18**. The second coating composition **24** can be applied to the luminal surface **16** and side surface **18** of the struts by contacting those surfaces with the substrate having the second coating composition **24** coated thereon to transfer the second coating composition **24** to the luminal surface **16** and side surface **18** of the struts.

Also, it may be preferable to mask or cover the surface that is not to be coated with a particular coating composition. For instance, as in **Figure 5a**, to avoid having the first coating composition disposed upon the luminal surface **16** and side surfaces 18 of the strut, the luminal surface 16 and side surfaces **18** can be masked.

The luminal surface **16** and side surfaces **18** can be masked, for instance, by application of a protective wrap to that surface. The protective wrap is a material that would protect the coated surface from exposure to the coating applied to the opposing surface. Suitable material for this protective wrap include, for example, PTFE film, dyna-leap, Kapton®, or any other appropriate type of covering or wrapping material. The protective wrap serves to protect the luminal surface **16** and side surfaces **18** from exposure to the coating composition as it is being applied to the abluminal surface **14**. Thus, the protective wrap will protect a luminal surface **16** and side surfaces **18** that has been already coated from additional deposition of the coating to be applied to the abluminal surface **14**. After the abluminal surface **14** of the struts **12** of the medical device have been coated, the wrap covering the luminal surface **16** and side surfaces **18** may be removed.

In one embodiment where only the luminal surface **16** is to be masked, the luminal surface **16** can be masked by placing the stent on a mandrel. The luminal surface **16** which is placed against the mandrel will not be exposed to a coating composition that is applied to the abluminal surface **14**. For example, in an embodiment, the stent that is mounted on the mandrel may then be rolled over a substrate containing a coating composition to transfer the coating composition to the abluminal surface **14** and side surfaces **18**. Alternatively, the stent can be placed on the mandrel and the abluminal surface **14** and side surfaces **18** of the strut are spray-coated with the coating composition. In yet another embodiment, it is possible to coat all surfaces of the stents, and remove the coating from desired areas by methods such as laser ablation.

For spray coating, a nozzle assembly may be used to spray a coating composition onto the inner surface. The nozzle assembly may be in the form of a cone that sprays the coating composition at an angle. The angle of the spray from the nozzles may need to be adjusted to ensure uniform thickness of the coating on the inner surface. Also, a nozzle assembly with small spray nozzles can be inserted into one end of the stent and moved through the stent until it extends past the opposite end of the stent Preferably, the spray mist flow is started while the nozzle is still outside of the stent. This step places a coating composition on the inside surface and one side surface of the struts of the stent The coating process may be repeated again. Preferably, the spray nozzle is inserted into the other end of the stent to coat the other side surface of the struts. By repeating the spraying from two directions, both side surfaces are coated with a coating composition.

In preferred embodiments of the invention, there is more than one coating composition applied to the stent In one embodiment, the second coating composition is see of the therapeutic agent when applied to the first coating composition. In other embodiments, the second coating composition is free of any therapeutic agent In still other embodiments, the second coating composition can comprise a therapeutic agent In some embodiments, the first coating composition is in the form of a first coating layer and the second coating composition is in the form of a second coating layer.

During the process of coating, the coating thickness can be controlled. In certain embodiments, the first coating layer has a thickness of about - I micrometer to about 20 micrometer. In other embodiments, the second coating layer has a thickness of about - 0.5 micrometer to about - 20 micrometer. In particular embodiments, the second coating layer has a thickness of less than 5 µm. In preferred embodiments, the first coating composition and second coating composition are coated to conform to the surfaces of the strut to preserve the openings of the sidewall structure.

After a coating composition has been applied, it can be cured. Curing is defined as the process of converting the polymeric material into the finished or useful state by the application of heat, vacuum, and/or chemical agents which induce physico-chemical changes. The applicable time and temperature, for curing are determined by the particular polymer involved and particular therapeutic agent used, if any, as known by one skilled in the art The coated medical devices may thereafter be subjected to a post-cum process wherein the medical devices are exposed to a low energy for stabilization of the coating. Also, after the medical device is coated, it preferably should be sterilized by methods of sterilization as known in the art.

In use, a coated medical device, such as an expandable stent, according to the present invention can be made to provide desired release profile of the therapeutic agent The medical devices and stents of the present invention may be used for any appropriate medical procedure. Delivery of the medical device can be accomplished using methods well known to those skilled in the art, such as mounting the stem on an inflatable balloon disposed at the distal end of a delivery catheter.

### E. Method of Making Porous Sarface

In embodiments of the stent of the present invention where the abluminal, luminal and/or side surfaces of the stent comprise a metal oxide or metal material with a plurality of pores, such as in **Figure 6a** and **6b**, the pores in some instances can be created by micro-roughing techniques involving the use of reactive plasmas, electrolyte etching or ion bombardment. The pores can also be created by other methods such as sand blasting, laser ablating or chemical etching.

In embodiments where the stent comprise a coating of a metal oxide or metal material having a plurality of pores or a cavity, such a coating can be formed in a number of ways. In some instances, the coating can be formed by depositing the material in a particular manner so that pores form in the material, for example using a sol-gel method - or, the metal oxide or metal material can be made porous by a deposition process such as sputtering and adjusting the deposition condition. Deposition conditions that can be adjusted or varied include, but are not limited to, chamber pressure, substrate temperature, substrate bias, substrate orientation, sputter rate, or a combination thereof

In an alternative method, the coating having a plurality of pores may be formed on the surface of the stent using vacuum plasma spraying of a spray composition comprising a metal oxide or metal under certain process parameters that promote the formation of pores.

In addition, the porous coating of metal oxide or metal material can be formed by a co-deposition technique. In such a technique the metal oxide or metal material is combined with a secondary phase material to form a composition. The secondary phase material can be a metal, such as carbon, aluminum, nickel or a non-metal. Preferably non-metal secondary materials include polymers that are capable of being leached off, such as polystyrene. The secondary phase material can be in the form of particles such as hollow spheres or chopped tubes of various sizes. The size of the pores formed will be determined by the size of the secondary phase material used. For example, if a hollow sphere of a second metal used as the secondary phase material, the size of the spheres will determine the size of the pores formed.

In some embodiments, the composition can contain a metal used to form the porous coating and a metal that is used as the secondary phase material. The two metals can form an alloy such as a gold/silver alloy, where gold is the metal used to form the porous coating and silver is the secondary phase material. Also, the two metals can be in the form of a mixture or a composite. As discussed below, the secondary phase material is removed to form the pores in the coating. Thus, if two metals are used in the composition, the metals should have different chemical or physical properties to facilitate removal of the metal that is used as the secondary phase material. For example, the metal that will be removed should be more electrochemically active. e.g., less corrosion-resistant than the metal used to form the porous coating. In some embodiments, the metal that will be removed should have a lower melting point than the metal used to form the porous coating. In yet another embodiment, the metal that will be removed should have a higher vapor pressure than the metal used to form the coating. Also, in another embodiment, the metal that is removed is more susceptible to being dissolved in a chosen solvent than the metal used to form the coating.

The composition containing the metal oxide or metal material is combined with a secondary phase material is applied to the surface of the medical device. Suitable application methods include but are not limited to, dipping, spraying, painting, electroplating, evaporation, plasma-vapor deposition, cathodic-arc deposition, sputtering, ion implantation, electrostatically, electroplating, electrochemically, a combination of the above, or the like.

Afterwards, the secondary phase material is removed from the composition to form a porous coating. For example, the secondary phase material may be removed from the composition by a dealloying process such as selective dissolution of the secondary phase material. In this method, the composition is exposed to an acid which removes the secondary phase material. Thus, the metal oxide or metal used to form the coating is preferably one that will not dissolve when exposed to the acid, while the secondary phase material is one that will dissolve in the acid. Any suitable acid can be used to remove the secondary phase material. One of ordinary skill in the art would recognize the appropriate concentration and reaction conditions to use. For example, if the secondary phase material is silver, nitric acid may be used at a concentration of up to 35% and a temperature up to 120°F. Also, a nitric acid and sulfuric acid mixture (95%/5%) immersion art 80°F may be used. The reaction conditions may be varied to vary the geometry, distribution, and depth of the coating.

Alternatively, the second metal can be removed anodically. For example, when silver is used as the secondary phase material, the silver may be removed from the composition applied to the surface anodically using a dilute nitric acid bath comprising up to 15% nitric acid, wherein the anode is the medical device, and the cathode comprises platinum. Voltages up to 10V DC can be applied across the electrodes. The bath chemistry, temperature, applied voltage, and process time may be varied to vary the geometry, distribution, and depth of the coating.

Furthermore, if the secondary phase material has a lower melting point than the metal oxide or metal used in the porous coating, the device coated with the composition containing the metal oxide or metal and the secondary phase material can be heated to a temperature such that the secondary phase material becomes a liquid and is removable from the metal oxide or metal. Examples of suitable metals for the porous coating include one of the higher melting point first metals: platinum, gold, stainless steel, titanium, tantalum, and iridium, in combination with a lower melting point secondary phase material such as: aluminum, barium, and bismuth.

In another embodiment, the secondary phase material has a higher vapor pressure than the metal oxide or metal used to form the porous coating. When the composition applied to the surface of the medical device is heated under vacuum the secondary phase material becomes vaporized and is removed from the metal oxide or metal.

A therapeutic agent is deposited in the pores of the metal oxide or metal material by any suitable method, such as, but not limited to dip coating, spray coating, spin coating, plasma deposition, condensation, electrochemically, electrostatically, evaporation, plasma vapor deposition, cathodic arc deposition, sputtering, ion implantation, or use of a fluidized bed. In order to dispose the molecules of the therapeutic agent in the pores, it may be necessary to modify the size of the pores in the coating or in the surface and outer region of the medical device. The pore size may be modified by any suitable method, such as heat treatment. If a polymer is also deposited in the pores, the polymer can be combined with the therapeutic agent and optionally a solvent A composition containing the polymer and therapeutic agent can be deposited in the pores. Alternatively, the polymer and therapeutic agent can be deposited in the pores separately.

The coating composition can be applied to the porous metal oxide or metal material by any method Examples of suitable methods include, but are not limited to, spraying such as by conventional nozzle or ultrasonic nozzle, dipping, rolling, electrostatic deposition, and a batch process such as air suspension, pan coating or ultrasonic mist spraying. Also, more than one coating method can be used. To facilitate the application of the polymer to the porous metal oxide or metal material, the polymer can be dispersed or dissolved in a solvent. After the coating composition comprising the solvent and the polymer is applied, the solvent is removed. Pores can be formed in the polymer by bubbling gas through the polymer, or by adding a second phase material to the solvent and polymer composition and dissolving the second phase material. In addition, a therapeutic agent can be loaded into the pores of the polymer by methods described above for loading a therapeutic agent into the pores of the metal oxide or metal material.

### F. Methods ot Making Cavities in Stent Struts

Cavities within struts designed to contain therapeutic agent can be created by techniques well known in the art for creating pores. For example, as embodied in **Figure 7a**-7c, cavities within stent struts can be made by laser ablation, micro-roughing techniques, sand blasting or chemical etchings or a combination of these methods. The cavities may also be formed by EDM (Electro Discharge Machine) microdrilling or electron beam drilling. The cavity created can be of any shape, size, and depth depending on need

There are a number of techniques to filling a cavity once it has been created. The stent can be submerged in a solution of drug, and then subsequently capped with a biodegradable coating composition to prevent the drug from leaking. The drug can be loaded onto the stent while submerged either by capillary action of the solution or by pressurizing the solution resulting in the drug driven into stent cavities. It is also possible to spray the drug into the cavity before adding the coating compositions. Examples of suitable methods include, but are not limited to, spraying such as by conventional nozzle or ultrasonic nozzle, dipping, rolling, electrostatic deposition, and a batch process such as air suspension, pen coating, or ultrasonic mist spraying. Also, more than one coating method can be used. To facilitate the application of the coating composition on the cavity, the coating composition can be dispersed or dissolved in a solvent. After the coating composition comprising the solvent and the polymer is applied, the solvent is removed.

The description contained herein is for purposes of illustration and not for purposes of limitation. Changes and modifications may be made to the embodiments of the description and still be within the scope of the invention. Furthermore, obvious changes, modifications or variations will occur to those skilled in the art. Also, all references cited above are incorporated herein, in their entirety, for all purposes related to this disclosure.

### FURTHER STENTS ARE GIVEN BELOW:

1. A stent designed for implantation into a blood vessel of a patient comprising:
   a tubular stent sidewall structure comprising a plurality of struts and openings in the sidewall structure; wherein at least one strut comprises an abluminal surface, and a luminal surface opposite the abluminal surface;
   a first coating composition comprising a first polymer and a therapeutic agent disposed on the abluminal surface and luminal surface; and
   a second coating composition disposed on a portion of the first coating composition that is disposed on the luminal surface wherein the second coating composition is biodegradable and comprises a bioabsorbable polymer.
2. The stent of paragraph 1, wherein the abluminal surface is free of the second coating composition; and wherein when the stent is implanted, the first coating composition disposed on the abluminal surface is in direct contact with the blood vessel.
3. The stent of paragraph 1, further comprising the second coating composition disposed on a portion of the first coating composition that is disposed on the abluminal surface.
4. The stent of paragraph 1, wherein the strut further comprises a first side surface and a second side surface opposite the first side surface, in which each side surface is disposed between the abluminal and luminal surfaces.
5. The stent of paragraph 4, wherein the first coating composition is disposed on the first and second side surfaces of the strut; and the second coating composition is disposed on a portion of the first coating composition that is disposed on each of the first and second side surfaces.
6. The stent of paragraph 1, wherein the first polymer is biostable.
7. The stent of paragraph 1, wherein the therapeutic agent comprises an anti-thrombogenic agent, anti-angiogensis agent, anti-proliferative agent, anti-restenosis agent, growth factor, antibiotic or radiochemical.
8. The stent of paragraph 1, wherein the therapeutic agent comprises an agent that inhibits smooth muscle cell proliferation.
9. The stent of paragraph 1, wherein the therapeutic agent comprises paclitaxel, sirolimus, everolimus, pimecrolimus or tacrolimus.
10. The stent of paragraph 1, wherein the second coating composition is free of the therapeutic agent when applied to the first coating composition.
11. The stent of paragraph 1, wherein the bioabsorbable polymer comprises a polyelectrolyte component.
12. The stent of paragraph 1, wherein the first coating composition and second coating composition conform to the surfaces of the strut to preserve the openings of the sidewall structure.
13. The stent of paragraph 1, wherein the stent is an intravascular stent.
14. An intravascular stent designed for implantation into a blood vessel of a patient comprising:
   a tubular stent sidewall structure, comprising a plurality of struts and openings in the sidewall structure; wherein at least one strut comprises an abluminal surface, and a luminal surface opposite the abluminal surface; a first side surface and a second side surface opposite the first side surface, in which each side surface is disposed between the abluminal and luminal surfaces;
   a first coating composition comprising a first biostable polymer and an anti-restenosis agent disposed on the abluminal surface; the luminal surface and side surfaces;
   a second coating composition disposed on the luminal surface, and on the first side surface and second side surface, wherein the second coating composition is biodegradable and comprises a bioabsorbable polymer; and
   wherein the abluminal surface is free of the second coating composition; and wherein when the stent is implanted, the first coating composition disposed on the abluminal surface is in direct contact with the blood vessel.
15. An intravascular stent designed for implantation into a blood vessel of a patient comprising:
   a tubular stent sidewall structure, comprising a plurality of struts and openings in the sidewall structure; wherein at least one strut comprises an abluminal surface, and a luminal surface opposite the abluminal surface; a first side surface and a second side surface opposite the first side surface, in which each side surface is disposed between the abluminal and luminal surfaces;
   a first coating composition comprising a first polymer and a therapeutic agent disposed on the abluminal surface, wherein the first coating composition is not disposed on the luminal or side surfaces; and
   a second coating composition disposed on the luminal surface, and on the first side surface and second side surface and on a portion of the first coating composition that is disposed on the abluminal surface, wherein the second coating composition is biodegradable and comprises a bioabsorbable polymer.
16. The stent of paragraph 15, wherein the first polymer is biostable.
17. The stent of paragraph 15, wherein the therapeutic agent comprises an anti-thrombogenic agent, anti-angiogensis agent, anti-proliferative agent, anti-restenosis agent, growth factor, antibiotic or radiochemical.
18. The stent of paragraph 17 wherein the therapeutic agent comprises an agent that inhibits smooth muscle cell proliferation.
19. The stent of paragraph 15, wherein the therapeutic agent comprises paclitaxel, sirolimus, everolimus, pimecrolimus or tacrolimus.
20. The stent of paragraph 15, wherein the second coating composition is free of the therapeutic agent when applied to the first coating composition.
21. The stent of paragraph 15, wherein the bioabsorbable polymer comprises a polyelectrolyte component.
22. A bioabsorbable intravascular stent designed for implantation into a blood vessel of a patient comprising:
   a tubular stent sidewall structure comprising a plurality of struts and openings in the sidewall structure and wherein at least one strut comprises a bioabsorbable material, and wherein the strut comprises an abluminal surface, a luminal surface opposite the abluminal
   surface and a cavity within the strut that is in fluid communication with the abluminal surface and a therapeutic agent within the cavity.
23. The stent of paragraph 22, wherein the stent further comprises a first coating composition comprising a first bioabsorbable polymer disposed on the abluminal surface and luminal surface.
24. The stent of paragraph 22, further comprising a second bioabsorbable polymer within the cavity.
25. The stent of paragraph 22, wherein the therapeutic agent comprises an anti-thrombogenic agent, anti-angiogensis agent, ariti-proliferative agent, anti-restenosis agent, growth factor, antibiotic or radiochemical.
26. The stent of paragraph 25, wherein the therapeutic agent comprises an anti-restenosis agent.
27. The stent of paragraph 22, wherein the therapeutic agent comprises an agent that inhibits smooth muscle cell proliferation.
28. The stent of paragraph 22, wherein the therapeutic agent comprises paclitaxel, sirolimus, everolimus, pimecrolimus or tacrolimus.
29. The stent of paragraph 22, wherein the bioabsorbable polymer comprises a polyelectrolyte component.
30. The stent of paragraph 22, wherein the first coating composition and second coating composition conforms to the surfaces of the strut to preserve the openings of the sidewall structure.

## Claims

1. A stent designed for implantation into a blood vessel of a patient comprising:
a tubular stent sidewall structure comprising a plurality of struts (12) and openings in the sidewall structure; wherein at least one strut (12) comprises an abluminal surface (14), a luminal surface (16) opposite the abluminal surface (14), and side surfaces (18) extending between the luminal and abluminal surfaces;
a first coating composition (22) disposed on the abluminal surface (14) and luminal surface (16); and
a second coating composition (24) disposed on a portion of the first coating composition (22); **characterized in that**
the portion is disposed either
only on the abluminal surface (14) and side surfaces (18) or
only on the abluminal surface (14); and
when the stent is implanted, the second coating composition (24) disposed on the abluminal surface (14) faces the blood vessel.

2. The stent of claim 1, wherein the first coating composition (22) is disposed also on the side surfaces (18).

3. The stent of claim 1, wherein the portion of the first coating composition is disposed only on the abluminal surface (14).

4. The stent of claim 1, wherein the first coating composition (22) is disposed on at least a portion of the first and second side surfaces (18) of the strut (12).

5. The stent of claim 4, wherein the second coating composition (24) is disposed on a portion of the first coating composition (22) that is disposed on the abluminal surface (14) and each of the first and second side surfaces (18).

6. The stent of claim 1, wherein the first coating composition (22) comprises a first polymer, in particular a first biostable polymer

7. The stent of claim 1, wherein the second coating composition (24) comprises a therapeutic agent and a bioabsorbable polymer.

8. The stent of claim 7, wherein the therapeutic agent comprises an anti-thrombogenic agent, anti-angiogensis agent, anti-proliferative agent, anti-restenosis agent, growth factor, antibiotic or radiochemical.

9. The stent of claim 7, wherein the therapeutic agent comprises an agent that inhibits smooth muscle cell proliferation.

10. The stent of claim 7, wherein the therapeutic agent comprises paclitaxel, sirolimus, everolimus, pimecrolimus, or tacrolimus.

11. The stent of claim 7, wherein the bioabsorbable polymer comprises a polyelectrolyte component.

12. The stent of claim 1, wherein the first coating composition (22) and second-coating composition (24) conform to the surfaces of the strut (12) to preserve the openings of the side wall structure.

13. The stent of claim 1, wherein the second coating composition is biodegradable.

14. The stent of claim 13, wherein the first coating composition is biodegradable, wherein the first coating composition degrades at a slower rate than the second coating composition.

15. The stent of claim 1, wherein the stent is fabricated from metallic materials, ceramic materials, polymer materials, or a combination thereof.
